# EUROPEAN PATENT APPLICATION

(11) **EP 4 702 995 A1**
(43) Date of publication of application: **04.03.2026**
(21) Application number: 24796208.7
(22) Date of filing: 26.04.2024
(51) Int. Cl.: A61L 31/10, A61L 31/08, A61L 31/14, A61L 31/16

(54) **DRUG COATING AND MEDICAL DEVICE COMPRISING SAME, SYSTEM, AND PREPARATION METHOD**

(30) Priority: 27.04.2023 CN 202310477411; 25.08.2023 CN 202311083219
(71) Applicant: Beijing Salubris Medtech Co., Ltd., Beijing 102600 (CN)
(72) Inventor: WANG, Jing, Beijing 102600 (CN); LI, Liang, Beijing 102600 (CN)
(74) Representative: WSL Patentanwälte Partnerschaft mbB
(86) International application number: PCT/CN2024/089941
(87) International publication number: WO 2024/222834

(57) **Abstract**

The present invention provides a drug coating and a medical device comprising the drug coating, a system, and a preparation method. The drug coating comprises a non-degradable fluorinated polymer matrix and a drug dispersed therein. An inner layer and an outer layer which have different drug-loading concentrations are formed along the thickness direction of the drug coating, the inner layer has a drug-loading concentration not less than 10 wt%, the outer layer has a drug-loading concentration less than 10 wt%, and the thickness of the outer layer is not greater than 20 µm. The drug coating of the present invention can allow for the release of a long-term stable amount of a drug into a tissue wall in contact with the drug coating, so as to achieve a long-term therapeutic or preventive effect on diseases such as luminal stenosis.

## Description

### TECHNICAL FIELD

The present invention relates to the technical field of medical devices, specifically relates to a drug-loading device and a coating for achieving long-term drug release, and particularly to an implantable medical device for intraluminal delivery, such as a drug-eluting stent, and a related preparation method.

### BACKGROUND

A drug-eluting stent (DES) consists of three parts: a metal skeleton, a polymer coating, and anti-restenosis drugs carried thereon. Since the application of DES, the polymer coating has been an important component of DES, serving as a drug carrier, controlling drug release, and preventing intra-stent thrombosis and restenosis.

DES is currently classified into two main categories: permanent polymers or biodegradable polymers.

Biodegradable polymers mainly use materials such as polylactic acid. After DES implantation, they degrade into carbon dioxide and water in human tissue and blood environment. The coating degrades and gradually becomes thinner and eventually disappears as the drug is released. The release kinetics curve of the drug in the body is generally considered to be jointly affected by the degradation characteristics of the biodegradable polymer and the drug loading concentration.

Permanent polymers are mainly fluoropolymers, which are non-degradable in the human body and have good biocompatibility. The release of the loaded drugs is generally considered to be mainly determined by the drug concentration of the coating. Fluoropolymer coatings are widely used in applications with short-term (usually less than 30 days) drug release requirements, such as coronary DES. However, there is a lack of research and reports on their application in permanent polymer DES with long-term (for example, more than 30 days) drug release requirements. Compared with biodegradable polymer coatings, which can extend the effective drug release time by designing the degradation characteristics and appropriate drug loading of biodegradable polymers, the design of fluoropolymer coatings to achieve long-term effective drug release is more challenging: directly increasing the drug loading concentration of the coating to extend the drug release time is generally not considered feasible, because this leads to excessively high and rapid drug release in the early stage of drug release, resulting in drug waste or significant drug toxicity. In the middle and late stages of drug release, the amount of drug retained in the coating decreases or the drug loading concentration decreases, resulting in lower drug release and lower release rate, which is insufficient to achieve the required effective drug dose within the desired treatment cycle and cannot achieve long-term effective drug release.

A typical example of the demand for long-term DES is the treatment of peripheral arterial disease (PAD). PAD is a chronic disease mainly caused by atherosclerosis, Takayasu's Arteritis, etc., and is a systemic disease. PAD often manifests as stenosis of the lower and/or upper limb arteries and reduced blood flow, posing a great threat to human health. DES is expected to have broad application prospects in peripheral arterial occlusive lesions due to its good biocompatibility and ability to load drugs.

However, what is more challenging is that compared with other blood vessels such as coronary arteries, lower-extremity peripheral arteries are longer, have larger diameters, and have stronger blood flow impact. For example, the diameter of the epicardial coronary artery is generally in a range from 0.5 to 5 mm, and the blood flow velocity is in a range from 10 to 30 cm/s, while the diameter of the femoral artery is generally in a range from 6 to 9 mm, and the blood flow velocity is in a range from 70 to 90 cm/s. In order to achieve long-term and stable treatment effects on lower limb PAD, DES needs to load a larger amount of drug, which exacerbates the initial large-scale release of drugs in a short period of time (burst release), and in the medium and long-term release cycle of more than 30 days, more stringent requirements are placed on the uniformity of the drug coating and a larger cumulative drug release. Otherwise, it may be difficult to achieve long-term effective therapeutic drug release.

### SUMMARY OF THE INVENTION

One of the objects of the present invention is to provide a medical device containing a drug coating, which utilizes the drug coating to release drugs to the tissue wall in contact with the device to achieve a therapeutic or preventive effect.

The drug coating comprises a non-degradable polymer as a coating matrix and a drug carrier. The drug can be dispersedly distributed in the coating matrix and released outside the coating during the treatment cycle, while the coating matrix is basically retained.

The present invention particularly proposes a drug coating or a medical device having the drug coating, wherein the drug coating comprises a non-degradable polymer matrix and a drug dispersed therein, and an outer layer and an inner layer with different drug loading concentrations are formed along the thickness direction of the drug coating, the inner layer has a drug loading concentration greater than that of the outer layer. The outer layer has a relatively small thickness, for example no greater than 20 µm. In a preferred embodiment, the thickness of the inner layer is greater than the thickness of the outer layer. The drug coating can be coated on the surface of the body of the medical device.

One of the objects of the present invention is to provide a method for preparing the aforementioned medical device containing a drug coating, the method comprises: firstly, coating a body of the medical device with a first coating composition to form an inner layer of the drug coating, and then coating the inner layer with a second coating composition to form an outer layer of the drug coating, wherein the drug loading concentration of the inner layer is greater than the drug loading concentration of the outer layer.

It should be understood that the inner layer and outer layer referred to in the present invention are continuous along the thickness direction of the coating, with no visible isolation, gap or interlayer between the inner layer and outer layer, and the inner layer is closer to the body than the outer layer. Typically, the inner layer and outer layer are composed of the same non-degradable polymer matrix.

As mentioned above, during the coating preparation process, the inner layer may be prepared first, and then the outer layer may be prepared on the surface of the inner layer. In certain embodiments, the inner layer is formed by coating a first coating composition in liquid form and then drying it, and the outer layer is formed by coating a second coating composition in liquid form on the outer side of the inner layer and then drying it. And the weight of the drug in the first coating composition is greater than the weight of the drug in the second coating composition.

The coating method can be any method known in the art, including but not limited to spray coating and dipping.

During the coating preparation process, it is also possible to choose to coat a base layer on the surface of the body of the device before coating the inner layer on the surface of the body of the device, that is, the base layer membrane-forming material and solvent is firstly mixed and dissolved to obtain a base layer solution, and then the base layer solution is coated on the surface of the body to form the base layer of the drug coating, so as to make the drug coating have better adhesion to the body, or prevent the drug in the drug coating from migrating to the surface of the body of the device. In one embodiment, the base layer membrane-forming material may be poly-n-butyl acrylate, and the solvent may be tetrahydrofuran.

In another aspect, the present invention also provides a drug-eluting stent having a diameter greater than 5 mm, particularly a drug-eluting stent suitable for peripheral arteries, which can provide uses such as preventing or treating stenosis and restenosis in large-diameter blood vessels, treating inflammation, and in particular providing long-term and stable drug release for more than 30 days.

As mentioned above, after a blood vessel with a large diameter lumen, such as a peripheral artery, is expanded by a drug-eluting stent at a narrowed site to expand the blood flow channel, the drug-eluting stent will be in a long-term impact environment of a large flow of blood. The present invention provides the aforementioned drug-eluting stent, which can withstand the long-term impact of a large flow of blood, avoid the early excessive release of drugs from the drug coating, and has the ability to release drugs continuously and stably in multiple smaller cycles within a longer treatment cycle (e.g., more than 30 days, or even about 1 year), so as to achieve long-term treatment or prevention effects.

In certain embodiments, the drug coating of the drug-eluting stent has a drug loading of not less than 100 µg/cm², such as 110 µg/cm², 120 µg/cm², or 130 µg/cm², or 140 µg/cm². In certain embodiments, the drug coating of the drug-eluting stent has a drug loading of not less than 150 µg/cm², such as 160 µg/cm², or 180 µg/cm², or 200 µg/cm². In certain embodiments, the drug coating of the drug-eluting stent has a drug loading of not less than 200 µg/cm², such as 210 µg/cm², or 230 µg/cm², or 250 µg/cm², or 260 µg/cm², or 280 µg/cm². In certain embodiments, the drug coating of the drug-eluting stent has a drug loading of not less than 300 µg/cm², such as 310 µg/cm², or 330 µg/cm², or 350 µg/cm², or 380 µg/cm² or 400 µg/cm². In certain embodiments, the drug coating of the drug-eluting stent has a drug loading of not less than 400 µg/cm², such as 410 µg/cm², or 450 µg/cm², or 480 µg/cm², or 500 µg/cm².

In certain embodiments, the drug coating thickness of the drug-eluting stent does not exceed 50 µm, and preferably, the drug coating thickness does not exceed 30 µm, for example, 30 µm, 25 µm, 20 µm, 15 µm or 12 µm.

Although it is a common practice for those skilled in the art to regulate the drug loading concentration, coating thickness and/or drug loading of a drug-loaded coating to control drug release, it has not been clearly anticipated by those skilled in the art that a drug coating that can be clinically used for long-term drug release can be obtained by this common practice. The inventors surprisingly discovered that, based on the control of the thickness of the inner and outer layers and/or drug loading concentration of the drug coating of the present invention under specific conditions, long-term controlled drug release from the non-degradable polymer drug-loaded coating can be achieved, especially clinically safe and therapeutic controlled drug release for greater than or far exceeding 30 days, such as meeting the needs of PAD treatment.

With respect to the drug coating of the present invention, the drug loading concentration of the inner layer is greater than that of the outer layer. At the same time, the thickness of the outer layer should not be too large, for example, not greater than 20 µm, but generally not greater than 10 µm. Particularly preferably, the thickness of the outer layer is not greater than 8 µm, for example, 6 µm, 5 µm, 4 µm, 3 µm, 2 µm, 1 µm.

Preferably, the thickness of the outer layer is in a range from 1 µm to 8 µm, particularly from 1 µm to 3 µm, or from 2 µm to 4 µm.

Based on the aforementioned drug coating, it is possible to achieve a significant reduction in the initial drug release under high drug loading conditions or avoid large-scale and concentrated drug release within a short period of time (e.g., 24 hours, 48 hours, or 7 days). This avoids the potential waste of drugs or dose toxicity to tissues caused by excessive initial drug release, and maintains a basically stable drug release over a longer release cycle, in order to achieve long-term therapeutic effects.

The present invention also provides a drug coating, in certain embodiments of which the drug coating is configured to release no greater than 30% of the drug in the first 24 hours, 48 hours, or 7 days after implantation into a mammalian blood vessel, and to release no less than 15% of the drug in the first 30 days.

The mammals may be humans, pigs, sheep, rabbits, etc.

The drug coating with the aforementioned drug release characteristics may be configured with any of the aforementioned selected settings of drug loading concentration and thickness of the inner and outer layers.

The inventors believe that the small drug loading and thin thickness of the outer layer may be the main reasons for achieving the above-mentioned effects. The inventors speculate that in the initial release stage of the drug coating, on the one hand, the low drug loading concentration of the outer layer directly affects the drug release amount of the drug coating in the initial release stage, and on the other hand, the non-degradable polymer matrix of the outer layer forms a shielding or blocking effect, delaying the release of the inner layer drug with a high drug loading concentration from the coating; and after the initial release stage of the drug coating, unexpectedly, the outer layer, from which most of the original drug may have been released, does not have properties similar to those of a blank non-degradable polymer matrix layer without drug loading, which almost completely blocks the release of the inner layer drug from the drug coating. At this time, the outer layer of the embodiment of the present invention forms a function similar to a control valve, and the drug loaded in the inner layer can maintain a stable amount of passage through the outer layer and migrate or diffuse to the outside of the drug coating, thereby realizing a stable amount of drug release from the drug coating to the outside (such as the tissue wall) over a long period of time. In certain embodiments, the drug release profile over a long period can be substantially linear.

Despite this speculation, those skilled in the art will appreciate that the controlled release mechanism of a coating is often complex, affected by multiple factors such as the swelling and dissolution of the coating matrix material in the tissue or blood environment, and the solubility, lipophilicity, or hydrophilicity of the drug.

In a preferred embodiment of the present invention, the thickness of the inner layer should be greater than that of the outer layer, and the thickness of the inner layer is generally not less than 10 µm. For example, the thickness can be selected to be 10 µm to 22 µm. In certain embodiments, the thickness of the inner layer is selected in the range of 12 µm to 16 µm.

For example, the thickness of the outer layer can be selected to be 1 µm, 2 µm, 3 µm, 4 µm, 5 µm, 6 µm, 7 µm, 8 µm, while the thickness of the inner layer can be selected to be 12 µm, 13 µm, 14 µm, 15 µm, 16 µm, 17 µm, 18 µm.

The thickness of the inner layer is selected to be greater than that of the outer layer, which is conducive to obtaining a drug loading concentration in the inner layer greater than that in the outer layer.

The drug loading concentration referred to in the present invention is expressed as a percentage by weight, and the drug loading concentration can be calculated as follows:
drug loading concentration of the inner layer = the mass of the drug contained in the inner layer / (the sum of the mass of the non-degradable polymer in the inner layer and the mass of the drug contained in the inner layer) × 100%; and
drug loading concentration of the outer layer = mass of the drug contained in the outer layer / (the sum of the mass of the non-degradable polymer in the outer layer and the mass of the drug contained in the outer layer) × 100%.

In an embodiment of the present invention, the inner layer and the outer layer of the drug coating are generally loaded with a total drug loading of not less than the total therapeutic amount required by the medical device. For treatment lumens with large diameters and lengths, the total drug loading of the drug coating can be greater than 100 µg/cm². For example, the total drug loading can be in a range from 110 µg/cm² to 400 µg/cm². Moreover, the drug loadings of the inner layer and the outer layer are usually different. Since the drug loading concentration of the inner layer is greater than that of the outer layer, or the thickness of the inner layer is greater than that of the outer layer, the drug loading of the inner layer is usually significantly greater than that of the outer layer. For example, the drug loading of the inner layer is in a range from 100 µg/cm² to 400 µg/cm², and the drug loading of the outer layer is in a range from 2 µg/cm² to 60 µg/cm²; or the drug loading of the inner layer is in a range from 150 µg/cm² to 350 µg/cm², and the drug loading of the outer layer is in a range from 2 µg/cm² to 30 µg/cm².

In some preferred embodiments of the present invention, the inner layer has a drug loading concentration of not less than 10 wt%, and the outer layer has a drug loading concentration of less than 10 wt%. The thickness of the outer layer can be selected to be not greater than 10 µm.

For example, in certain embodiments, the inner layer has a drug loading concentration ranging from 10 wt% to 45 wt%, and the outer layer has a drug loading concentration ranging from 1 wt% to 8 wt%.

In certain embodiments, the inner layer has a drug loading concentration ranging from 10 wt% to 30 wt%, and the outer layer has a drug loading concentration ranging from 3 wt% to 5 wt%.

In certain embodiments, the inner layer has a drug loading concentration ranging from 10 wt% to 20 wt%, and the outer layer has a drug loading concentration ranging from 3 wt% to 5 wt%.

In certain embodiments, the inner layer has a drug loading concentration ranging from 10 wt% to 20 wt%, and the outer layer has a drug loading concentration ranging from 3 wt% to 4 wt%.

In certain embodiments, the inner layer has a drug loading concentration ranging from 20 wt% to 30 wt%, and the outer layer has a drug loading concentration ranging from 4 wt% to 5 wt%;

In certain embodiments, the inner layer has a drug loading concentration ranging from 15 wt% to 25 wt%, and the outer layer has a drug loading concentration ranging from 3 wt% to 5 wt%.

In certain embodiments, the inner layer has a drug loading concentration of 10 wt%, 13 wt%, 15 wt%, 18 wt% or 20 wt%, and the outer layer has a drug loading concentration of 3 wt%, 4 wt% or 5 wt%.

Another object of the present invention is to provide a drug coating having a specific drug release profile or a medical device containing the drug coating and a treatment method, wherein the release profile of the drug coating satisfies the requirement that the cumulative drug release within the first 24 hours after implantation into blood vessels of a mammal is not greater than 40%, and the cumulative drug release within the first 30 days is not less than 15%.

In certain embodiments, the cumulative drug release of the drug coating within the first 30 days after implantation into blood vessels of a mammal is not less than 15%, and not greater than 60%, not greater than 50%, or not greater than 40%. Preferably, the cumulative drug release is not greater than 40%.

In certain embodiments, the cumulative drug release of the drug coating within the first 14 days after implantation into blood vessels of a mammal is not less than 15%, and not greater than 30%, not greater than 40%, or not greater than 50%. Preferably, the cumulative drug release is not greater than 30%.

In certain embodiments, the weekly cumulative drug release of the drug coating within the second week to the seventeenth week after implantation into blood vessels of a mammal is in a range from 6% to 2%.

In certain embodiments, the cumulative drug release of the drug coating within the 31^{st} to 360^{th} day after implantation into blood vessels of a mammal is not less than 30%, or not less than 40%, or not less than 50%. Preferably, the cumulative drug release is not less than 50%.

In certain embodiments, the cumulative drug release of the drug coating within the first 24 hours after implantation into blood vessels of a mammal is not greater than 15%, or not greater than 20%, or not greater than 30%. Preferably, the cumulative drug release within the first 24 hours is not greater than 15%.

The non-degradable polymer referred to in the present invention has biocompatibility and chemical and physical stability, and is suitable for contact with tissue walls of the human body or animal body, such as blood vessels. Preferred non-degradable polymers may be fluoropolymers. Those skilled in the art understand that fluoropolymers refer to polymers with a C-C chain as the main chain and one or more fluorine atoms, or even all fluorine atoms, attached to the side chains or branched chains.

Fluoropolymers can be selected from the fluorine-containing copolymers, and the comonomers can be, for example, tetrafluoroethylene (TFE), hexafluoropropylene, chlorotrifluoroethylene and perfluoroalkyl vinyl ether (PFVE), among which PFVE includes perfluoromethyl vinyl ether (methyl ether), perfluoropropyl vinyl ether (propyl ether) and perfluorodioxole.

Fluoropolymers may also preferably be fluorine-containing acrylic polymers, for example, comonomers may be perfluoroalkyl (meth)acrylates, heteroatom-containing perfluoroalkyl (meth)acrylates, perfluoroamide (meth)acrylates, and perfluorosulfonamide (meth)acrylates.

The fluorine-containing copolymer used in the present invention as a membrane-forming polymer should have a molecular weight high enough to provide sufficient toughness so that the membrane containing such polymer will not be rubbed off during the stent processing or installation process, or the coating will not show obvious cracking when the stent or other medical devices such as occluders and vena cava filters expand.

In a preferred embodiment, the fluoropolymer is a copolymer of polyvinylidene fluoride and hexafluoropropylene (PVDF-HFP), and the copolymer comprises 50% to 92% of vinylidene fluoride and 50% to 8% of hexafluoropropylene by weight. The polymer has been reported and used, for example, in Chinese invention patent publication number CN1225292C, the full text of which is incorporated into the present specification. The Chinese invention patent discloses the preparation method and performance testing of the aforementioned fluorine-containing polymer coating.

In certain embodiments, the non-degradable fluoropolymer is a copolymer of vinylidene fluoride and hexafluoropropylene, and the copolymer comprises 55% to 65% of vinylidene fluoride and 45% to 35% of hexafluoropropylene by weight.

In certain embodiments, the non-degradable fluoropolymer is a copolymer of vinylidene fluoride and hexafluoropropylene, and the copolymer comprises 70% to 90% of vinylidene fluoride and 30% to 10% of hexafluoropropylene by weight.

In certain embodiments, the drug coating further includes a base layer without drug loading, and the base layer is located below the inner layer and covers the body of the medical device, and is used to allow the drug coating to attach to the body of the medical device. In this case, the inner layer is not directly attached to the surface of the body of the medical device.

The base layer can be made of a material selected from the group consisting of polymethacrylonitrile, polymethyl methacrylate, polyethyl methacrylate, polypropyl methacrylate, poly-n-butyl methacrylate, polyhydroxyethyl methacrylate, and polyhydroxypropyl methacrylate. The base layer also has good toughness, so the coating will not crack.

Another object of the present invention is to provide an implantable medical system for interventional procedures, comprising the above-mentioned medical device and a delivery device detachable from the medical device, wherein the body of the medical device or at least a portion thereof has expandable properties and is suitable for being compressed to a smaller diameter and delivered through a catheter. The aforementioned drug coating may be provided on the surface of the expandable portion of the medical device. Therefore, the drug coating preferably has good elongation, and satisfies the requirement that the drug coating remains substantially intact without cracking when the medical device is deformed from a compressed state to an unconstrained state.

In certain embodiments, the medical device may be a drug-eluting stent, and the stent may be crimped to 50% of its unconstrained diameter while the drug coating remains essentially intact with no cracking.

In certain embodiments, the medical device may be a drug-eluting stent, and the stent may be crimped to 30% of its unconstrained diameter while the drug coating remains essentially intact with no cracking.

In certain embodiments, the medical device may be a drug-eluting stent, and the stent may be crimped to 20% of its unconstrained diameter while the drug coating remains essentially intact with no cracking.

Drug-eluting stents typically have a body made of metal, such as stainless steel, nickel-titanium alloy, or cobalt-chromium alloy. The drug coating is arranged on the alloy material surface of the body.

Based on some embodiments of the present invention, it can be expected that the provided drug coating has the function of meeting the requirements of long-term stable drug release. In particular, some embodiments provide long-term and stable drug release for more than 30 days, 60 days, 180 days or 360 days. The drug release amount during the release cycle can be always controlled above the minimum effective dose to ensure or improve the medical effect.

### BRIEF DESCRIPTION OF THE DRAWINGS

FIG. 1 is a schematic structural diagram of a drug-eluting stent according to an example of the present invention;
FIG. 2 is a schematic cross-sectional view of a slender rod portion of a drug-eluting stent according to an example of the present invention;
FIG. 3 shows the cumulative drug release results of the drug-eluting stent according to an example of the present invention in animal experiments;
FIG. 4 shows the drug release rate results of the drug-eluting stent according to an example of the present invention in animal experiments;
FIG. 5 shows the cumulative drug release results of the drug-eluting stent according to an example of the present invention in an in vitro test;
FIG. 6 is a schematic structural diagram of an implantable medical device system for interventional procedures according to an example of the present invention;
FIG. 7 is a partial enlarged view of FIG. 6, showing the assembled position of the drug-eluting stent in the delivery device.

### DETAILED DESCRIPTION

The present invention will be further described in the following examples. However, these examples are merely exemplary and are intended to help those skilled in the art understand the present invention rather than to limit the scope of protection of the present invention. In addition to the examples, there may be other variations in different forms, and it is not necessary to list all implementation methods here. It can be understood that all equivalent changes or modifications made according to the spirit of the present invention are included in the scope of protection of the present invention.

The present invention provides one or more examples of a drug-eluting stent having a drug coating attached to the surface of the stent. The drug coating may be any of the following or those described above in the specification.

The drug-eluting stent is mainly used to treat diseases within the vascular cavity, such as stenosis, restenosis, and inflammation of peripheral arteries, peripheral veins, cerebral nervescerebrovascular and neurovascular, and arteriovenous fistulas. However, those skilled in the art will appreciate that the drug-eluting stent can also be used to treat diseases related to the esophagus, intestines, and other body cavities.

Without limitation, the drug coating can also be applied to other medical devices, such as various occluders, filters, etc. that can be permanently or temporarily placed in the body through interventional procedures, or medical devices that require long-term contact with tissue walls without interventional procedures. The drug coating is attached to the surface of the device body in order to enable the drug coating to deliver drugs outward.

The present invention provides some examples of the drug coating having long-term controlled-release functional properties, such as a drug release profile that delivers drugs to the tissue wall for 30 days or more, or 60 days or more, or 120 days or more, or 240 days or more, or 360 days or more, while satisfying that the average daily release amount or the average weekly release amount of the drug within the preset release cycle at least reaches or exceeds the required minimum therapeutic dose.

In some examples, the minimum therapeutic dose required is 1% to 10% of the total drug loading of the drug coating. In some examples, the drug release profile satisfies an average weekly release of at least 1% to 10% of the total drug loading of the drug coating, such as 8%, 6%, or at least 1% to 5%, such as 1%, 2%, 3%, or 4%, of the total drug loading of the drug coating.

In some examples, in contrast to the aforementioned average weekly release, the drug release profile satisfies the requirement that the weekly designed release of the drug within the preset release cycle (for example, 4 to 52 weeks) at least reaches or exceeds the required minimum therapeutic dose, which is 1% to 10% of the total drug loading of the drug coating. In some examples, the drug release profile satisfies the preset release cycle, wherein the designed weekly release of the drug reaches or exceeds at least 1% to 6%, for example 1%, 2%, 3%, 4%, 5%, or 6%, of the total drug loading of the drug coating.

Compared with the average weekly release, the designed weekly release can more reasonably reflect the characteristics of long-term drug release control, especially the long-term release control requirements of the minimum therapeutic dose, because the average weekly release may not be sufficient to truly reflect or reflect the control of the actual weekly drug release. The designed weekly release refers to the amount of controlled release drug required in each week from the first week to the last week within the preset drug release cycle. In order to ensure long-term therapeutic effects, the designed weekly release amount must at least reach or exceed the required minimum therapeutic dose.

The present invention further provides another examples of the drug coating, which has the functional characteristic of inhibiting the short-term burst release of high drug loading drug coatings, for example, the high drug loading coating has a cumulative drug release of not greater than 40% within the first 24 hours of drug delivery to the tissue wall, and a cumulative drug release of not less than 15% within the first 30 days. Unless otherwise specified, the cumulative drug release referred to above, here and below in the present invention means the ratio of the amount of drug released from the drug coating to the total amount of drug loaded in the drug coating.

In some examples, the high drug loading coating has a cumulative drug release of not greater than 30%, or not greater than 20%, or not greater than 10% within the first 24 hours of drug delivery to the tissue wall, and at the same time, the cumulative drug release within the first 30 days is not less than 15%, or not less than 20%, or not less than 30%, or not less than 40%.

It has been proven that applying a barrier layer to the drug coating is particularly helpful in inhibiting the short-term burst release of drugs from the drug coating. The barrier layer can be an additional coating layer that is the same as or different from the drug coating matrix. The barrier layer usually does not load drugs and can be called a blank barrier layer. However, the blank barrier layer of the non-degradable polymer matrix makes it difficult for the drug coating underneath to release drugs to the outside of the coating, and it may not be possible to maintain the minimum therapeutic dose of drug release for a long time during the release cycle. Therefore, one direction for improvement is to select a matrix material that can be rapidly broken and dissolved as a sacrificial barrier layer to block drug release before drug release or in the early stage of the release cycle, and to achieve the removal of the barrier layer during the release cycle, for example, by dissolution, degradation, fluid flushing, etc.

Different from the above, the drug coating of the example of the present invention mainly realizes short-term and long-term drug release control through a double-layer drug-loading layer with gradient concentration, especially for drug coatings with high drug loading.

Compared with coronary drug-eluting stents used in small-diameter blood vessels, which usually have a drug loading of 10 µg/cm², the high drug loading drug coating referred to in the present invention can usually have a drug loading of not less than 50 µg/cm², especially not less than 100 µg/cm².

In certain preferred examples of the present invention, the aforementioned long-acting controlled release functional characteristics and the functional characteristics of inhibiting short-term burst release of high drug loading coatings are simultaneously satisfied.

In some examples, the cumulative drug release of the drug coating within the first 30 days after implantation into blood vessels of a mammal is not less than 15%, and not greater than 60%, not greater than 50%, or not greater than 40%.

In some examples, the cumulative drug release of the drug coating within the first 14 days after implantation into blood vessels of a mammal is not less than 15%, and not greater than 30%, not greater than 40%, or not greater than 50%.

In some examples, the weekly cumulative drug release of the drug coating within the second week to the seventeenth week after implantation into blood vessels of a mammal is in a range from 6% to 2%.

In some examples, the cumulative drug release of the drug coating within the 31^{st} to 360^{th} day after implantation into blood vessels of a mammal is not less than 30%, or not less than 40%, or not less than 50%.

In some examples, the cumulative drug release of the drug coating within the first 24 hours after implantation into blood vessels of a mammal is not greater than 15%, or not greater than 20%, or not greater than 30%.

A significant feature of the drug coating of the example of the present invention is that it provides a thinner outer layer and a markedly thicker inner layer. Typically, the outer layer has a lower drug loading and/or drug loading concentration, while the inner layer has a higher drug loading and/or drug loading concentration.

In one example, the thickness of the outer layer is selected from the range of 1 µm to 6 µm, while the thickness of the inner layer having a significant thickness is selected from the range of 10 µm to 18 µm.

The present invention further provides an example of a drug-eluting stent having the aforementioned drug coating, which is particularly suitable for implantation into a peripheral artery.

Certain drug-eluting stent examples suitable for implantation in peripheral arteries may have a length of 15 mm to 200 mm and a diameter of 3 mm to 12 mm. In some examples, the drug-eluting stent may have a length L of 20 mm to 150 mm and a diameter of 5 mm to 10 mm.

In one example, the drug-eluting stent has a length of 20 mm and a diameter of 5 mm. In another example, the drug-eluting stent has a length of 150 mm and a diameter of 8 mm. Both can be delivered through a sheath with an outer diameter of 7F.

The drug coating may be applied to the entire surface of the body of the stent, but is not limited thereto. Those skilled in the art may also choose to apply the drug coating to part of the body of the stent. For example, some parts of the body of the stent may have an exposed surface that is not coated with the drug coating.

The body of the stent is usually made of metal and has good elastic deformation characteristics to provide good support and crimping performance.

A significant advantage of the drug-eluting stent implanted in a peripheral artery of the present invention is that it provides a less damaging and more effective treatment effect during the peripheral artery treatment process.

The drug-eluting stent can be delivered to the diseased site in the blood vessel by a delivery device when it is crimped to a smaller diameter. The delivery device is operated to release the drug-eluting stent into the blood vessel lumen. The drug-eluting stent can self-expand or be expanded by a balloon, and contact the blood vessel wall and use its radial force to support the expansion of the narrowed blood vessel wall. The drug carried by the coating, such as an anti-proliferative drug, can be released to the diseased site to prevent and reduce restenosis of the blood vessel lumen.

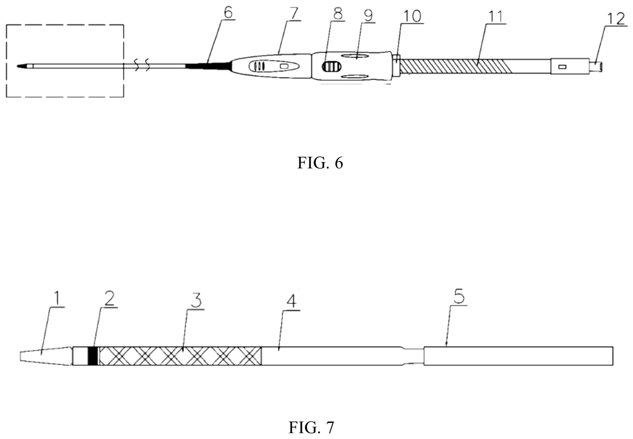

In one example, as shown in FIG. 6 and FIG. 7, the drug-eluting stent 3 can be assembled into a delivery device. Referring to FIG. 7, the drug-eluting stent 3 is sleeved on the inner sheath 4 of the delivery device, and an outer sheath 5 is slidably sleeved on the outer side of the inner sheath 4 to restrain the drug-eluting stent 3 on the inner sheath 4. The distal end 1 of the inner sheath 4 is a conical head, and a radiopaque ring 2 is provided near the distal end 1. The proximal end of the inner sheath 4 is connected to the front handle 7, and a stress diffusion tube 6 is provided at the distal end of the front handle 7 to improve the strength of the tube body. During the operation, the delivery device injects physiological saline through the proximal connector 12 to flush the tube body, and is delivered into the human body through the introduction channel established by the guide wire. When the distal end of the inner sheath 4 reaches the designated lesion location, the operator removes the limit of the knob 8 by the stopper 10, holds the front handle 7, releases the lock between the button 9 and the screw 11, and retracts the operating knob 8 along the screw 11 (moves toward the proximal end), driving the outer sheath 5 to retract to release the drug-eluting stent 3. FIG. 7 shows that the outer sheath 5 slides toward the proximal end to expose the drug-eluting stent 3, so that the drug-eluting stent 3 is no longer constrained by the outer sheath 5. During the release process, the self-expanding stent is no longer subject to radial constraints of the outer sheath 5 from the distal end to the proximal end, and thus gradually expands and tends to restore its preset shape to support the expansion of the narrowed blood vessel lumen within the blood vessel.

Some drug-eluting stent examples have the capability of inhibiting the burst release of drugs that occurs in the early stages of stent implantation, which is very advantageous in most cases.

For example, for drug coatings loaded with rapamycin or its analogs, in the early postoperative period of angioplasty, platelet adhesion, aggregation, and secretion may occur due to damage to endothelial cells caused by stent expansion, tearing and exposure of the vascular endothelium, reduction of the vascular endothelial barrier and protective function, vascular stretching, and impact of blood flow. At this time, the released drugs such as rapamycin may have no significant effect. Therefore, the amount of drug released should not be too large. For drug coatings loaded with paclitaxel, etc., in the early postoperative period of angioplasty, for the same reasons mentioned above, a large amount of paclitaxel released in a short period of time may exhibit severe cytotoxicity, causing additional damage to the vascular wall tissue or other tissues in the systemic circulation.

Although studies have shown that the incidence of restenosis in peripheral arteries such as the superficial femoral artery is high within one year after dilation, see Osamu et al., "Timing of the restenosis following nitinol stenting in the superficial femoral artery and the factors associated with early and late restenoses", published in Catheterization & Cardiovascular Interventions Official Journal of the Society for Cardiac Angiography & Interventions in 2011, no suitable peripheral arterial drug-eluting stent has been proposed to address this clinical problem.

Therefore, an object of the present invention is to provide a method for preventing or treating restenosis and a drug-eluting stent used in the method.

The drug-eluting stent may be any of the examples described above or below, and satisfies the requirement that, in one example, the drug-eluting arterial stent implanted in a stenotic peripheral artery has a drug release cycle of more than 3 months.

In another example, a drug-eluting arterial stent implanted in a stenotic peripheral artery has a drug release cycle exceeding 6 months.

In a preferred example, the drug-eluting arterial stent implanted in a stenotic area of a peripheral artery has a drug release cycle of approximately 12 months. This is because, considering that the aforementioned studies show that the restenosis time has a steep peak at about 12 months, therefore, in order to effectively reduce the restenosis rate, the entire drug release cycle of the drug-eluting stent is designed to be one year. The drug that inhibits stenosis can maintain a long-term effective dose release, so as to more effectively improve the 12-month patency rate of the lumen, especially maintaining the effective dose release of the drug in the period before the 12^{th} month of stent implantation when the probability of restenosis is higher, which may have a better effect in preventing or reducing restenosis. In some examples, the amount of drug released by the drug-eluting stent in the 7^{th} to 12^{th} month after implantation is 10% to 40%, such as approximately 30%, of the total drug loading of the drug coating.

The following is an example of selecting polyvinylidene fluoride copolymer (PVDF-HFP) as the fluoropolymer matrix for drug coating. However, it is not limited thereto, and those skilled in the art may also select other suitable fluoropolymer matrices.

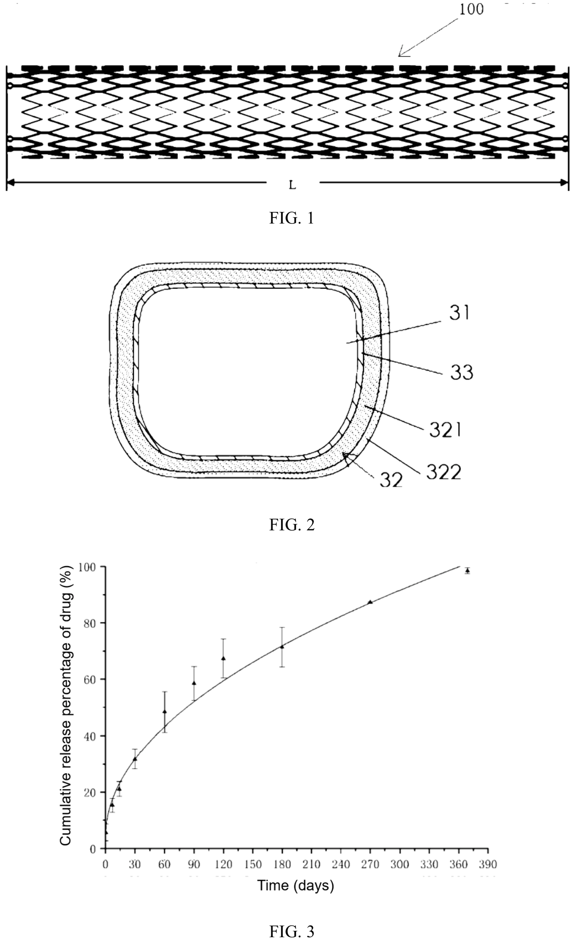

Referring to FIG. 1, FIG. 1 shows an example of a drug-eluting stent 100 having a cylindrical structure formed by a mesh body, and can provide radial support to the lumen of a blood vessel.

Referring to FIG. 2, FIG. 2 shows a drug coating 32 covering the surface of the body 1 of a drug-eluting stent 100 or other similar device. The drug coating 32 may substantially surround the body 1 in the circumferential direction. The figure shows that the cross section of the slender rod portion of the body 1 of the stent is a quadrilateral. However, in some examples, the cross section can also be a circular, elliptical or arc-shaped structure.

Without being limiting, the drug coating does not necessarily need to circumferentially surround the body 1. In some examples, the drug coating may only cover an outer peripheral surface of the body 1 of the stent, that is, the surface of the body 1 that contacts the tissue wall. In some examples, the outer peripheral surface of the body 1 of the stent may be provided with a groove, and the drug coating may selectively cover only the groove or cover the entire outer peripheral surface of the body 1 of the stent at the same time.

In some examples, the outer peripheral surface and an inner peripheral surface of the body 1 of the stent may be covered with drug coatings of the same or different thicknesses. In some examples, the thickness of the drug coating layer covering the outer peripheral surface of the body 1 of the stent is greater than the thickness of the drug coating layer covering the inner peripheral surface.

The body 1 of the stent can be made of non-biodegradable materials, especially metals, such as 316L stainless steel, cobalt-chromium alloy, magnesium alloy, iron alloy, zinc alloy, nickel-titanium alloy, nickel-iron alloy and other biocompatible metals with good memory properties and support. However, the material of the body 1 of the stent is not limited thereto, and the body 1 of the stent may also be a polymer, such as polyamide, polyolefin, non-absorbable polyester, and the like. Without being limited thereto, the body 1 of the stent may also be made of a biodegradable or bioabsorbable material and substantially or mostly not degraded or absorbed during the drug release cycle, although this option may not be preferred.

The drug coating matrix of the example of FIG. 1 is a polyvinylidene fluoride copolymer (PVDF-HFP), which is copolymerized by vinylidene fluoride and hexafluoropropylene. The polymer has excellent biocompatibility, and its stable chemical properties ensure stable drug release without the risk of producing acidic degradation substances that increase inflammatory responses. The drugs loaded in the drug coating can be anti-proliferative drugs such as rapamycin and its derivatives, paclitaxel, and heparin. In the example of FIG. 1, the loaded drug is selected as rapamycin, which has the effects of inhibiting cell proliferation and migration. Rapamycin also has the effects of reducing the production of local vascular wall cytokines and the activation of inflammatory cells, inhibiting cell apoptosis, and promoting re-endothelialization of vascular injury sites. Restenosis is mainly caused by excessive proliferation of the vascular endothelium after stent implantation. Rapamycin inhibits cell proliferation and migration, thereby inhibiting the occurrence of restenosis.

The drug coating 32 of the example of FIG. 2 comprises an inner layer 321 with a polyvinylidene fluoride copolymer as a matrix, and an outer layer 322 also with a polyvinylidene fluoride copolymer as a matrix. The concentrations of rapamycin loaded in the inner layer 321 and the outer layer 322 are different, wherein the drug loading concentration of the inner layer 321 is greater than that of the outer layer 322. FIG. 2 shows that the thickness of the inner layer 321 is greater than the thickness of the outer layer 322. The thickness of the outer layer 322 should not be too large, for example, not greater than 20 µm, but generally not greater than 10 µm. In this example, the thickness of the outer layer 322 is in a range from 1 µm to 8 µm. The overall thickness of the inner layer 321 and the outer layer 322 is generally not greater than 50 µm, and preferably not greater than 30 µm.

In some examples, the overall thickness of the inner layer 321 and the outer layer 322 may be selected to be 11 µm to 27 µm.

For example, the thickness of the inner layer 321 is in a range from 10µm to 22µm, and the thickness of the outer layer 322 is in a range from 1µm to 5µm. The thickness of the inner layer 321 can be twice or more than the thickness of the outer layer 322. For example, the coating thickness ratio of the inner layer 321 to the outer layer 322 is about in a range from 22:1 to 10:1. For another example, the thickness of the inner layer 321 is in a range from 12 µm to 16 µm, the thickness of the outer layer 322 is in a range from 1 µm to 3 µm, and the coating thickness ratio of the inner layer 321 to the outer layer 322 is about in a range from 16:1 to 12:1; or, the thickness of the inner layer 321 is in a range from 12 µm to 16 µm, the thickness of the outer layer 322 is in a range from 2 µm to 4 µm, and the coating thickness ratio of the inner layer 321 to the outer layer 322 is about in a range from 16:2 to 12:2.

The drug coating 32 of the example of FIG. 1 further comprises a base layer 33 that does not load a drug. Although this is not essential, it is preferred in some cases to comprise the base layer 33. The inner layer 321 of the drug coating 32 is attached to the surface of the base layer 33, and the base layer 33 is directly attached to the surface of the body 31 of the stent. Selecting a suitable base layer 33 can enhance the adhesion of the drug coating 32 on the stent. The material of the base layer 33 can be, for example, non-biodegradable polymers such as polymethacrylonitrile, polymethyl methacrylate, polyethyl methacrylate, polypropyl methacrylate, polybutyl methacrylate, polyhydroxyethyl methacrylate, and polyhydroxypropyl methacrylate, which basically will not cause tissue rejection and tissue inflammation due to degradation. In this example, polybutyl methacrylate (PBMA) is used as the base layer 33. PBMA has strong adhesion to the metal material of the body 31 of the stent and good compatibility with the drug-loaded fluorine-containing copolymer. This allows the contact interface between the inner layer 321 and the base layer 33 to intermingle, thereby "locking" each other to form a stable and high-strength coating system on the stent.

The thickness of the base layer 33 should not be too large, which can be selected by those skilled in the art according to needs. In this example, the thickness of the base layer 33 is in a range from 0.5 µm to 1.5 µm, for example, 0.5 µm, 1 µm, or 1.5 µm.

It can be understood that the drug molecules carried in the inner layer 321 close to the surface of the body 31 of the stent must migrate a longer distance to be released outside the coating. At the same time, as the drug is released, the drug concentration difference between the drug-eluting stent and the blood or tissue gradually decreases, and the release gradually becomes slower.

Therefore, in this example, along the coating thickness direction, the drug concentration of at least part of the inner layer 321 is greater than the drug concentration of at least part of the outer layer 322, forming a double-layer drug coating 32 with a gradient concentration. Obviously, in a preferred example, the inner layer 321 and the outer layer 322 each have a relatively uniform drug concentration, and the drug concentration of the entire area of the inner layer 321 is greater than the drug concentration of the entire area of the outer layer 322, which is particularly beneficial for controlling the release of the entire drug coating 32.

In this example, the drug loading concentration of the inner layer 321 may be 10 wt% to 45 wt%, and the drug loading concentration of the outer layer 322 may be 1 wt% to 8 wt%. Preferably, the drug loading concentration of the inner layer 321 can be selected to be 10 wt% to 30 wt%, and the drug loading concentration of the outer layer 322 can be 3 wt% to 5 wt%; or the drug loading concentration of the inner layer 321 can be 10 wt% to 20 wt%, and the drug loading concentration of the outer layer 322 can be 3 wt% to 5 wt%; or the drug loading concentration of the inner layer 321 can be 10 wt% to 20 wt%, and the drug loading concentration of the outer layer 322 can be 3 wt% to 4 wt%. More preferably, the drug loading concentration of the inner layer 321 can be selected to be 10 wt%, 13 wt%, 15 wt%, 18 wt% or 20 wt%, and the drug loading concentration of the outer layer 322 can be selected to be 3 wt%, 4 wt% or 5 wt%.

In one example of the present invention, the cumulative drug release of the drug-eluting stent 12 months after implantation is greater than or equal to 98%, with little burst release in the initial release and long-term release maintained in the middle and late stages.

The drug coating 32 of this example is loaded with a sufficient amount of drug, for example, the drug concentration can be selected to be in a range from 110 µg/cm² to 500 µg/cm².

Obviously, the above-mentioned drug-eluting stent examples are not limited to peripheral arteries, but can also be applied to other arteries or veins with fast blood flow or thick blood vessels that require long-term release.

The following provides an example of preparing the aforementioned drug-eluting stent.

### Example 1

A method for preparing a drug-eluting stent comprises the following steps:
1) providing a mesh tube-shaped body of the stent, ultrasonically cleaning and drying it;
2) preparing a base layer coating material without drug loading, including:
   The base layer membrane-forming material and the solvent are mixed and dissolved to obtain a base layer solution. Specifically, the base layer solution is prepared by weighing 0.40 g of poly(n-butyl acrylate), the mixture is added into a 250 ml volumetric flask, 60 ml of tetrahydrofuran is added into the volumetric flask, and the mixture is stirred until the mixture is completely dissolved.
3) forming a base layer on the stent body, including:
   The base layer solution is loaded into the ultrasonic spraying equipment (ultrasonic atomization spraying system TLS-2000). After the body of the stent being cleaned and dried, the body of the stent is placed under ultrasonic spraying until the liquid flowed out of the nozzle. The base layer with a thickness of 0.5 to 1.5 µm is sprayed. After the spraying being completed, it is dried in an oven at 50°C for 2 hours.
4) preparing a drug-loaded coating material, specifically including:
   4 g of PVDF-HFP (75%-25%) solid is weighed, and added into a 50 ml volumetric flask, methyl ethyl ketone is added to the volume, stirred until completely dissolved to obtain the carrier stock solution, and stored below room temperature.
   5 ml of each of the above carrier stock solutions are taken and added them into a 100 ml volumetric flask, 16.5 mg and 100 mg of rapamycin solid are added respectively, made it up to volume with methyl ethyl ketone, and stirred until completely dissolved. These are used as the coating material solutions for preparing the outer and inner layers of the drug coating.
5) forming a drug-loaded coating on the base layer of the stent, including:
   After the base layer being formed, the drug-loaded coating layer is sprayed on the base layer to form the inner layer and the outer layer. Firstly, spraying the inner layer: 1 mg/mL of inner layer coating material solution is loaded into an ultrasonic spraying device (ultrasonic atomization spraying system TLS-2000). After the liquid flowed out of the nozzle, the stent is placed under ultrasonic spraying and sprayed to form an inner layer with a thickness of 12 to 16 µm. After spraying, the stent is dried in a vacuum oven at 40°C for 12 hours.

Then spraying the outer layer: 0.165 mg/mL of outer layer coating material solution is loaded into the aforementioned ultrasonic spray equipment until the liquid flows out of the nozzle, the stent is placed under ultrasonic spraying, the stent is sprayed to form an outer layer with a thickness of 2 to 4 µm. After spraying, it is dried in a vacuum oven at 40°C for 12 hours.

Through the above steps, the drug-eluting stent of this example is obtained, wherein the drug loading concentration of the inner layer is 20 wt%, the drug loading concentration of the outer layer is 4 wt%, the drug loading of the inner layer is 200 µg/cm² to 268 µg/cm², the drug loading of the outer layer is 7 µg/cm² to 14 µg/cm², the thickness of the inner layer is 12 µm to 16 µm, and the thickness of the outer layer is 2 µm to 4 µm.

In other examples, the drug-eluting stent may not include a base layer. After the body of the stent is cleaned and dried, the inner layer and outer layer solutions are sprayed in sequence to form corresponding inner and outer layers, so that the drug coating can directly attach to the surface of the body of the stent through the inner layer.

Obviously, the steps of this example are not limited to the above-described exemplary sequence. For example, a base layer solution, an inner layer solution, or an outer layer solution may be prepared simultaneously, and then the corresponding solution may be selected and sprayed on the body of the stent as needed.

### Example 2

With reference to the preparation method of Example 1, this example prepares a drug-eluting stent having a different drug loading concentration than that of Example 1.

The base layer in this example is the same as that in Example 1 and will not be described again here.

The coating material solution preparation method for the inner layer and outer layer of the drug coating is as follows:
9 g of PVDF-HFP (50%-50%) solid is weighed, and added them into a 50 ml volumetric flask, methyl ethyl ketone is added to make up the volume, stirred until completely dissolved to obtain the carrier stock solution, and stored below room temperature.

1 ml of each of the above-mentioned carrier stock solutions are taken and put them into a 100 ml volumetric flask, 6 mg and 20 mg of rapamycin solid are added respectively, made up to volume with methyl ethyl ketone, and stirred until completely dissolved. These are used as the coating material solutions for preparing the outer and inner layers of the drug coating.

Firstly, the inner layer is sprayed. 20 mg/mL of the inner layer coating material solution is loaded into an ultrasonic spraying device (ultrasonic atomization spraying system TLS-2000). After the liquid flowed out of the nozzle, the stent is placed under the ultrasonic spraying. The inner layer is sprayed on the base layer to form a thickness of 14-18 µm. After spraying, the stent is dried in a vacuum oven at 40°C for 12 hours.

Then spraying the outer layer: 6 mg/mL of outer layer coating material solution is loaded into the aforementioned ultrasonic spray equipment until the liquid flows out of the nozzle and the stent is placed under ultrasonic spraying to form an outer layer with a thickness of 1 to 3 µm. After spraying, it is dried in a vacuum oven at 40°C for 12 hours.

Through the above steps, the drug-eluting stent of this example is obtained, wherein the inner layer drug loading concentration is 10 wt%, the outer layer drug loading concentration is 3 wt%, the drug loading of the inner layer is 126 µg/cm² to 162 µg/cm², the drug loading of the outer layer is 3µg/cm² to 9µg/cm², the thickness of the inner layer is 14 µm to 18 µm, and the thickness of the outer layer is 1 µm to 3 µm.

### Example 3

Referring to the preparation method of Example 1, this example prepares another drug-eluting stent having a different drug loading concentration compared with Examples 1 and 2. The base layer in this example is the same as that in example 1 and will not be described again here.

The coating material solution preparation method for the inner layer and outer layer of the drug coating is as follows:
5 g of PVDF-HFP (60%-40%) solid is weighed, and added into a 50 ml volumetric flask, methyl ethyl ketone is added to make up the volume, stirred until completely dissolved to obtain the carrier stock solution, and stored below room temperature.

1 ml of each of the above-mentioned carrier stock solutions are taken and put them into a 100 ml volumetric flask, 5.3 mg and 44 mg of rapamycin solid are added respectively, made up to volume with methyl ethyl ketone, and stirred until completely dissolved. These are used to prepare the outer layer coating material solution with a concentration of 5.3 µg/ml and the inner layer coating material solution with a concentration of 440 µg/mL.

Firstly, the inner layer is sprayed. 400 µg/mL of inner layer coating material solution is loaded into an ultrasonic spraying device (ultrasonic atomization spraying system TLS-2000). After the liquid flowed out of the nozzle, the stent is placed under the ultrasonic spraying. The inner layer with a thickness of 10 to 12 µm is sprayed on the base layer. After spraying, the stent is dried in a vacuum oven at 40°C for 12 hours.

Then spraying the outer layer: 21.5 µg/mL of outer layer coating material solution is loaded into the aforementioned ultrasonic spray equipment until the liquid flows out of the nozzle and the stent is placed under ultrasonic spraying to form an outer layer with a thickness of 3 to 5 µm. After spraying, it is dried in a vacuum oven at 40°C for 12h.

Through the above steps, the drug-eluting stent of this example is obtained, wherein the inner layer drug loading concentration is 30 wt%, the outer layer drug loading concentration is 5 wt%, the drug loading of the inner layer is 264 µg/cm² to 316 µg/cm², the drug loading of the outer layer is 13 µg/cm² to 22 µg/cm², the thickness of the inner layer is 10µm to 12µm, and the thickness of the outer layer is 3 µm to 5 µm.

### Animal Test Example

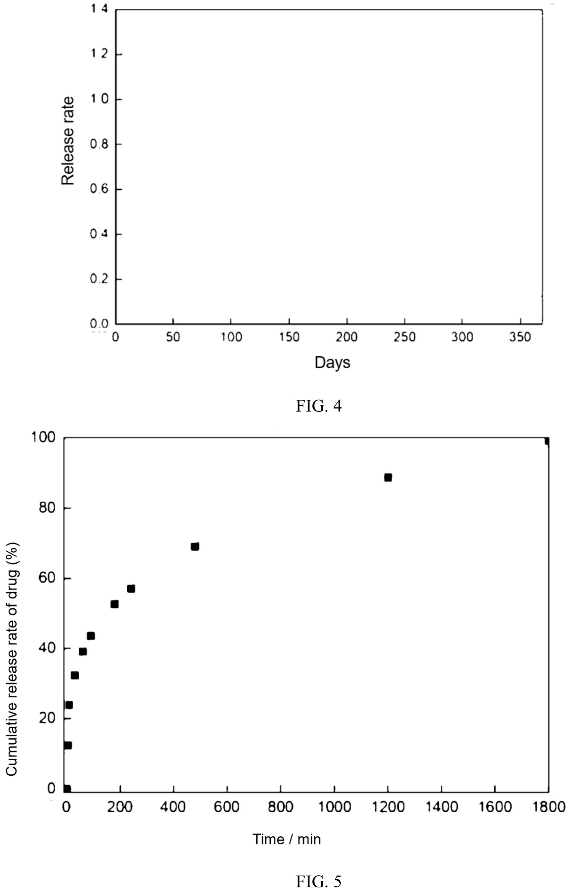

Using miniature Bama pigs as an animal model, 66 sets of drug-eluting stents from Example 1 were randomly distributed and implanted into 33 miniature pigs, with one set of drug-eluting stents implanted into each pig's left and right iliac (femoral) arteries. After implantation, two sets of stents implanted in three pigs were removed at each of the 11 time points, and the drug residue in the stent drug coating was analyzed by liquid chromatography. The average drug residue in the drug coating at each time point was measured and calculated. The cumulative drug release data of the drug-eluting stent in Example 1 at each time point were obtained, as shown in Table 1 below. Based on this, the cumulative drug release curve and release rate curve were plotted, as shown in FIGS. 3 and 4.

Still using the miniature Bama pig as the animal model, the six sets of drug-eluting stents in Example 2 were randomly implanted into three miniature pigs, with one set of drug-eluting stents implanted into each pig's left and right iliac (femoral) arteries. After implantation, two sets of stents implanted in one pig were removed at each of three time points, and the drug residue in the stent drug coating was analyzed by liquid chromatography. The average drug residue in the drug coating at each time point was measured and calculated, and the cumulative drug release data of the drug-eluting stent in Example 2 at each time point were obtained, as shown in Table 1 below.

Similarly, six sets of drug-eluting stents of Example 3 were randomly assigned and implanted into three miniature pigs, with one drug-eluting stent implanted into each pig's left and right iliac (femoral) arteries. After implantation, two sets of stents implanted in one pig were removed at each of three time points, and the drug residue in the stent drug coating was analyzed by liquid chromatography. The average drug residue in the drug coating at each time point was measured and calculated, and the cumulative drug release data of the drug-eluting stent in Example 3 at each time point were obtained, as shown in Table 1 below.

**Table 1: Cumulative drug release in miniature Bama pigs**

| Time | Example 1 | Example 2 | Example 3 |
|---|---|---|---|
| 4h | 5.70% | | 9.98% |
| 24h | 6.36% | 1.23% | - |
| 7d | 15.33% | - | |
| 14d | 21.13% | - | - |
| 30d | 31.72% | 24.88% | |
| 60d | 48.58% | - | 55.50% |
| 90d | 58.50% | - | |
| 120d | 67.31% | 60.56% | - |
| 180d | 71.34% | - | 88.61% |
| 270d | 87.24% | | |
| 369d | 98.52% | - | - |

According to the above data and accompanying drawings, it can be seen that the drug release of the drug coating of Example 1 covers the entire restenosis cycle (369 days) and the release rate is as high as over 90%. Within the first 1 to 2 months when smooth muscle proliferation is relatively rapid, the drug is released at a relatively fast rate. After about 50 days, the cumulative drug release enters a relatively plateau region and the drug maintains a stable release rate until the drug is completely released.

Based on the above data, the drug coatings of these examples have the following effects on the development of restenosis:
(1) Within 1 to 12 hours after angioplasty, platelets adhere, aggregate and secrete due to damage to endothelial cells caused by angioplasty, tearing and exposure of the vascular endothelium, reduction of endothelial barrier and protective function, vascular stretching and blood flow impact. At this time, rapamycin has no significant effect. Therefore, the release amount should not be too large. The drug-eluting stent of the above example controls the release rate to about 10% in the early stage, avoiding sudden release of drugs and ensuring that sufficient drugs are retained for release in the later stage.
(2) Immediately after surgery, which can last for about 7 days, is also caused by endothelial cell damage. At this stage, rapamycin mainly inhibits inflammation and has not yet exerted an anti-proliferative effect. Therefore, rapamycin has no significant effect at this stage. The drug-eluting stent of the above example controls the release rate to less than 20% at this stage, avoiding premature waste and loss of drugs.
(3) The peak of smooth muscle proliferation is reached after 7 to 14 days and can last for 1 to 2 months. Within two months, the peak of smooth muscle proliferation causes intimal thickening, leading to restenosis in the later stage. During this stage, the above example releases about 50% of the drug, that is, it reaches the peak stage to release enough drugs to meet the treatment needs while ensuring smooth and stable release during this stage.
(4) Finally, the blood vessels adapt to the changes in the external environment by self-adjusting to adapt to the changes in hemodynamics and vascular wall structure under different physiological and pathological conditions. In the middle and late stages, for example, at 270 days, the drug can still be continuously released, reaching a release rate of 80% or more, until the maximum release is completed at the time point of the entire release cycle.

### In Vitro Test Example

Despite the aforementioned animal studies, an in vitro accelerated test can usually be used first to roughly evaluate the release characteristics of the drug coating of the drug-eluting stent. In the in vitro accelerated release experiment, the release medium can be selected to have a solvent or a combination thereof that is more likely to release the drug than the blood, so as to obtain evaluation results of different drug coatings in a shorter period of time.

Therefore, the present invention also provides a method for in vitro drug release testing of a drug-eluting stent, comprising using a buffer solution system of a trace amount of a water-soluble nonionic surfactant mixed with acetonitrile as a release medium. For example, a buffer solution system containing about 0.1% to 1% of a water-soluble nonionic surfactant mixed with about 10% of acetonitrile is used as the release medium. The pH value of the release medium can be adjusted to around 7.4.

Taking the drug-eluting stent of Example 1 as an example, the steps of performing an in vitro drug release test were as follows:
1) Preparation of release medium: 20 mL of acetonitrile and 1.8 mL of 70% Triton X-405 were placed in a 200 mL volumetric flask, made up to volume with PBS solution (38.0 g of sodium dihydrogen phosphate and 5.04 g of disodium hydrogen phosphate, and water were added to make up to 1000 mL), shaken well, and the pH was adjusted to 7.4 ± 0.1 with hydrochloric acid or sodium hydroxide to obtain a release medium composed of 0.9% Triton X-405 mixed with 10% acetonitrile.
2) Perform release test: the drug-eluting stent to be tested was placed in a release medium, maintained it in a water bath at 37°C ± 2°C, an oscillation frequency of 60 r/min was selected to simulate drug release, and the drug-eluting stent was removed at time points of 5 min, 10 min, 30 min, 60 min, 90 min, 180 min, 240 min, 480 min, 1200 min, and 1440 min, then placed it in a dissolution bottle containing acetonitrile and sonicate in an ice bath for 30 min to completely dissolve the residual rapamycin in the drug-eluting stent in the acetonitrile in the dissolution bottle. After quantification, the dissolved drug in the dissolution bottle for detection by chromatography was measured.

The measured release curve is shown in FIG. 5. It can be seen that the release rate reached 90% or more at the late stage of 1440 minutes, and approached 100% at 1800 minutes.

Multiple stents of Example 1 were prepared, and one stent was taken out at each time point for drug dissolution testing.

Using the same preparation method as above, several other examples of drug-eluting stents with different drug coatings as shown in the following table were prepared.

| Example | Inner layer concentration wt% | Inner layer thickness µm | Outer layer concentration wt% | Outer layer thickness µm |
|---|---|---|---|---|
| 4 | 15 | 10 | 1 | 1 |
| 5 | 20 | 12 | 5 | 3 |
| 6 | 20 | 12 | 3 | 3 |
| 7 | 25 | 12 | 5 | 5 |
| 8 | 20 | 16 | 3 | 5 |

### Comparative Example

With reference to the method of Example 1, a drug-eluting stent of comparative example was prepared, comprising a base layer covering the body of the stent, a drug-loading layer covering the base layer, and a blank barrier layer covering the drug-loading layer. The base layer is the same as that in Example 1, and the drug-loading layer and the blank barrier layer are the same fluoropolymer as that in Example 1.

In the comparative example, the drug loading layer is a coating layer containing a single concentration of drug, the drug loading concentration is 10 wt% to 20 wt%, the thickness of the drug-loading layer is 12 µm to 15 µm, and the thickness of the barrier layer is 2 µm to 4 µm. The blank barrier layer is a fluoropolymer coating without drug loading.

By studying in vivo or in vitro drug release curve, the results show that the comparative example reduces the burst release phenomenon in the early stage of release, but the drug release in the middle and late stages is obviously insufficient, and the release rate over 30 days is far below 15%, making it difficult to achieve a therapeutic effect.

A drug-eluting stent without a barrier layer of comparative example is also prepared using the same or similar preparation method as above, including a base layer covering the body of the stent and a drug-loading layer covering the base layer, without a barrier layer or other drug-loading layer being provided on the drug-loading layer. The base layer is the same as that in Example 1, and the drug-loading layer is the same fluoropolymer as that in Example 1.

The drug-loading layer parameters were set as shown in the following table to prepare Comparative Examples 1-4.

| Comparative Example | Inner layer concentration wt% | Inner layer thickness µm | Outer layer concentration wt% | Outer layer thickness µm |
|---|---|---|---|---|
| **1** | **20** | **12** | **0** | **8** |
| **2** | **15** | **10** | **0** | **3** |
| **3** | **20** | **12** | **none** | **none** |
| **4** | **25** | **15** | **none** | **none** |

### In Vitro Release Comparison Experiment

Taking the drug-eluting stents of Examples 4-8 and Comparative Examples 1-4 as examples, in vitro drug release tests were performed.

To observe the release of drug-eluting stents over a longer release time, the release medium used in the in vitro drug release test was prepared using the following method: 8

| No. | Cumulative drug release | | | | | | | | |
|---|---|---|---|---|---|---|---|---|---|
| | 0.5h | 1.5h | 2.5h | 5h | 10h | 15h | 20h | 30h | 45h |
| Example4 | 21.16% | 27.39% | 30.75% | 41.66% | 52.86% | 60.92% | 66.89% | 82.94% | 96.08% |
| Example5 | 15.89% | 23.63% | 28.50% | 36.20% | 46.33% | 54.12% | 60.70% | 71.74% | 85.29% |
| Example6 | 11.84% | 23.53% | 27.31% | 38.59% | 44.11% | 50.45% | 55.50% | 63.50% | 72.66% |
| Example7 | 9.00% | 18.04% | 23.32% | 31.30% | 41.34% | 48.82% | 55.00% | 65.17% | 77.34% |
| Example8 | 10.78% | 21.75% | 27.54% | 35.07% | 43.82% | 49.24% | 54.01% | 60.27% | 67.65% |
| Comparative Example 1 | 8.88% | 13.96% | 17.72% | 20.43% | 26.84% | 29.24% | 30.75% | / | / |
| Comparative Example 2 | 11.84% | 19.18% | 23.53% | 26.75% | 29.54% | 34.00% | 38.59% | / | / |
| Comparative Example 3 | 38.43% | 39.43% | 44.73% | 45.47% | 63.68% | 74.58% | 84.29% | / | / |
| Comparative Example 4 | 27.40% | 36.95% | 40.60% | 45.14% | 54.68% | 72.64% | 79.87% | / | / |

mL of acetonitrile and 0.6 mL of Tween-20 were placed in a 200 mL volumetric flask, made up to volume with PBS solution (38.0 g of sodium dihydrogen phosphate and 5.04 g of disodium hydrogen phosphate, and water were added to make up to 1000 mL), shaken well, and the pH was adjusted to 7.4 ± 0.1 with hydrochloric acid or sodium hydroxide to obtain a release medium composed of 0.3% Tween-20 mixed with 4% acetonitrile.

The drug-eluting stent to be tested was placed in the release medium, maintained in a water bath at 37°C ± 2°C, and oscillated at a frequency of 60 r/min to simulate drug release. The stent was removed at multiple corresponding time points and placed in a dissolution bottle containing acetonitrile. Ice-bath sonication was performed for 30 minutes to completely dissolve the residual rapamycin in the drug-eluting stent in the acetonitrile in the dissolution bottle. After quantification, the amount of dissolved drug in the dissolution bottle was sampled and tested by chromatography. The corresponding drug release results are shown in the following table.

In each example and comparative example, multiple stents were prepared. In the aforementioned examples, one stent was taken out at each time point to perform drug dissolution testing.

At intermediate time points, such as 10 hours, the release rate of the present invention can reach 40%, and even Example 4 can reach 50% or more, which is significantly different from Comparative Examples 1 and 2, which can only reach a release rate of about 26% to 29%; and it is significantly different from Comparative Examples 3 and 4, which show a trend of burst release.

As can be seen from the above table, the drug-loading layers of Examples 4-8 can release the drug evenly and slowly, achieving long-term sustained release. Compared with Comparative Examples 2 and 3 without a barrier layer, there is no burst release in the initial stage. Compared with Comparative Example 1 with a blank barrier layer, the drug can be released in a stable amount for a long time, and the release rate in the later stage is significantly higher.

Although the present invention has been disclosed above in terms of preferred examples, it is not intended to limit the present invention. Any person skilled in the art may make possible changes and modifications to the technical solutions of the present invention by using the methods and technical contents disclosed above without departing from the spirit and scope of the present invention. Therefore, any simple modifications, equivalent changes and modifications made to the above examples based on the technical essence of the present invention without departing from the content of the technical solutions of the present invention shall fall within the scope of protection of the technical solutions of the present invention.

## Claims

1. A drug coating, wherein the drug coating is used to be provided on a medical device, the drug coating comprises a non-degradable fluoropolymer matrix and a drug dispersed therein, and an outer layer and an inner layer with different drug loading concentrations are formed along the thickness direction of the drug coating, the drug loading concentration of the inner layer is greater than the drug loading concentration of the outer layer, and the thickness of the outer layer is not greater than 20 µm.

2. The drug coating according to claim 1, wherein the drug coating is configured such that the cumulative drug release within the first 24 hours after implantation into blood vessels of a mammal is not greater than 40%, and the cumulative drug release within the first 30 days is not less than 15%.

3. The drug coating according to claim 1 or 2, wherein the mammal is a human or a pig.

4. The drug coating according to any one of claims 1 to 3, wherein the inner layer has a drug loading concentration of not less than 10 wt% by weight, and the outer layer has a drug loading concentration of less than 10 wt% by weight; or
the inner layer has a drug loading concentration ranging from 10 wt% to 45 wt%, and the outer layer has a drug loading concentration ranging from 1 wt% to 8 wt%; or
the inner layer has a drug loading concentration ranging from 10 wt% to 30 wt%, and the outer layer has a drug loading concentration ranging from 3 wt% to 5 wt%; or
the inner layer has a drug loading concentration ranging from 10 wt% to 20 wt%, and the outer layer has a drug loading concentration ranging from 3 wt% to 5 wt%; or
the inner layer has a drug loading concentration ranging from 10 wt% to 20 wt%, and the outer layer has a drug loading concentration ranging from 3 wt% to 4 wt%; or
the inner layer has a drug loading concentration ranging from 20 wt% to 30 wt%, and the outer layer has a drug loading concentration ranging from 4 wt% to 5 wt%; or
the inner layer has a drug loading concentration of 10 wt%, 13 wt%, 15 wt%, 18 wt% or 20 wt%, and the outer layer has a drug loading concentration of 3 wt%, 4 wt% or 5 wt%.

5. The drug coating according to any one of claims 1 to 4, wherein the outer layer has a thickness of not less than 1 µm and not greater than 10 µm, or the outer layer has a thickness ranging from 1 µm to 8 µm.

6. The drug coating according to any one of claims 1 to 5, wherein the inner layer has a thickness of greater than that of the outer layer, or the inner layer has a thickness ranging from 10 µm to 22 µm.

7. The drug coating according to any one of claims 1 to 6, wherein the inner layer has a thickness ranging from 12 µm to 16 µm, and the outer layer has a thickness ranging from 2 µm to 4 µm.

8. The drug coating according to any one of claims 1 to 7, wherein the total drug loading of the drug coating is in a range from 110 µg/cm² to 500 µg/cm².

9. The drug coating according to any one of claims 1 to 8, wherein the drug loading of the inner layer is greater than that of the outer layer.

10. The drug coating according to any one of claims 1 to 9, wherein the drug loading of the inner layer is in a range from 100 µg/cm² to 400 µg/cm², and the drug loading of the outer layer is in a range from 2 µg/cm² to 60 µg/cm²; or the drug loading of the inner layer is in a range from 150 µg/cm² to 350 µg/cm², and the drug loading of the outer layer is in a range from 2 µg/cm² to 30 µg/cm².

11. The drug coating according to any one of claims 1 to 10, wherein the drug is selected from a group consisting of paclitaxel, rapamycin, heparin, probucol, dexamethasone, and analogs thereof.

12. The drug coating according to any one of claims 1 to 11, wherein the drug is selected from lipophilic drugs.

13. The drug coating according to any one of claims 1 to 12, wherein the drug coating is configured such that the cumulative drug release within the first 30 days after implantation into blood vessels of a mammal is not less than 15%, and not greater than 60%, not greater than 50%, or not greater than 40%.

14. The drug coating according to any one of claims 1 to 13, wherein the drug coating is configured such that the cumulative drug release within the first 14 days after implantation into blood vessels of a mammal is not less than 10%, and not greater than 30%, not greater than 40%, or not greater than 50%.

15. The drug coating according to any one of claims 1 to 14, wherein the drug coating is configured such that the weekly cumulative drug release within the second week to the seventeenth week after implantation into blood vessels of a mammal is in a range from 6% to 2%; or
the drug coating is configured such that the cumulative drug release within the 31^{st} to 360^{th} day after implantation into blood vessels of a mammal is not less than 30%, or not less than 40%, or not less than 50%.

16. The drug coating according to any one of claims 1 to 15, wherein the drug coating is configured such that the cumulative drug release within the first 24 hours after implantation into blood vessels of a mammal is not greater than 15%, or not greater than 20%, or not greater than 30%.

17. The drug coating according to any one of claims 1 to 16, wherein the non-degradable fluoropolymer is a copolymer of vinylidene fluoride and hexafluoropropylene, and the copolymer contains 50% to 92% of vinylidene fluoride and 50% to 8% of hexafluoropropylene by weight.

18. The drug coating according to any one of claims 1 to 17, wherein the non-degradable fluoropolymer is a copolymer of vinylidene fluoride and hexafluoropropylene, and the copolymer contains 55% to 65% of vinylidene fluoride and 45% to 35% of hexafluoropropylene by weight.

19. The drug coating according to any one of claims 1 to 18, wherein the non-degradable fluoropolymer is a copolymer of vinylidene fluoride and hexafluoropropylene, and the copolymer contains 70% to 90% of vinylidene fluoride and 30% to 10% of hexafluoropropylene by weight.

20. The drug coating according to any one of claims 1 to 19, wherein the drug coating further comprises a base layer without drug loading, and the inner layer is attached to the base layer.

21. The drug coating according to any one of claims 1 to 20, wherein the base layer is made of one or more of polymethacrylonitrile, polymethyl methacrylate, polyethyl methacrylate, polypropyl methacrylate, polybutyl methacrylate, polyhydroxyethyl methacrylate, and polyhydroxypropyl methacrylate.

22. A medical device containing a drug coating, comprising a body of the medical device and the drug coating according to any one of claims 1 to 21, wherein the drug coating is provided on the surface of the body.

23. The medical device according to claim 22, wherein the body of the medical device has an expandable portion, and the drug coating is provided on a surface of the expandable portion.

24. The medical device according to claim 22 or 23, wherein the medical device is a drug-eluting stent, an occluder or a filter, and the medical device has a body made of metal.

25. The medical device according to any one of claims 22 to 24, wherein the drug coating is provided on the surface of a portion of the body having a diameter greater than or equal to 5 mm.

26. The medical device according to any one of claims 22 to 25, wherein the medical device is a drug-eluting stent for treating peripheral arterial stenosis, and the drug-eluting stent has a length of 15 mm to 200 mm and a diameter of 3 mm to 12 mm.

27. An implantable medical device system, comprising a medical device according to any one of claims 22 to 26 and a delivery device detachable from the medical device, wherein the medical device is installed in the delivery device in a compressed state during delivery, and when the medical device reaches the lesion location, the delivery device is detached from the medical device, so that the medical device contacts the tissue wall in an expanded state.

28. A method for preparing a medical device containing a drug coating, wherein the method is used to prepare the medical device according to any one of claims 22 to 26, and the method comprises:
providing a body of the medical device;
preparing a first solution and a second solution to form drug coatings having different drug loading concentrations;
firstly, coating the body with the first solution to form an inner layer of the drug coating;
then, coating the inner layer with the second solution to form an outer layer of the drug coating;
wherein the drug loading concentration of the first solution is greater than that of the second solution, and the thickness of the inner layer is greater than that of the outer layer.

29. The method according to claim 28, wherein the method further comprises:
preparing a base layer solution, wherein a base layer membrane-forming material and a solvent are mixed and dissolved to obtain the base layer solution;
coating the surface of the body with the base layer solution to form the base layer, and coating the body with the first solution on the base layer to form the inner layer of the drug coating.
